# EUROPEAN PATENT APPLICATION

(11) **EP 1 870 453 A1**
(43) Date of publication of application: **26.12.2007**
(21) Application number: 06729700.2
(22) Date of filing: 22.03.2006
(51) Int. Cl.: C12N 9/58, C12N 15/09, A23L 1/30, A61K 38/46, C12N 1/15, C12N 1/19, C12N 1/21, C12N 5/10, C12N 1/14, C12R 1/77

(54) **NOVEL PROTEASE, MICROORGANISM PRODUCING THE SAME, AND APPLICATION THEREOF**

(30) Priority: 22.03.2005 JP 2005082817
(71) Applicant: SODX CO., LTD., Kawachinagano-shi, Osaka, 5860071 (JP)
(72) Inventor: NAKAMURA, Takumi, Kawachinagano-shi, Osaka 5860044 (JP)
(74) Representative: HOFFMANN EITLE
(86) International application number: PCT/JP2006/305733
(87) International publication number: WO 2006/101140

(57) **Abstract**

An object of the present invention are to provide a protease that is stable in a wide pH range from acidic to alkaline, and that has excellent thrombolytic activity; a protease-producing microorganism that produces the above protease; and a process for producing the protease.

By culturing a novel filamentous fungus belonging to the genus *Fusarium* (*Fusarium* sp. strain BLB), a protease that is stable in a wide pH range from acidic to alkaline and that has excellent thrombolytic activity, is formed and accumulated in the culture medium, and recovered.

## Description

### TECHNICAL FIELD

The present invention relates to a novel protease that exhibits excellent thrombolytic activity, a protease-producing microorganism that produces the above protease, and a process for producing the protease. The present invention further relates to a thrombolytic agent or food containing the protease, and a fermented food produced using the protease-producing microorganism.

### BACKGROUND ART

Proteases are a group of enzymes that hydrolyze the peptide bonds of proteins and peptides. Various proteases derived from microorganisms, animals, and plants, have been developed and applied in the fields of medicines and foods. For example, proteases contained in tempeh or tempeh fungus reportedly have thrombolytic activity and are useful as thrombolytic agents (Patent Document 1).

However, almost none of the known proteases used in the fields of medicines and foods are stable in a wide pH range from acidic to alkaline. Proteases that are stable in a wide pH range are useful in applications such as foods, medicines, etc. In particular, proteases that are stable in a wide pH range and that have thrombolytic activity are highly useful as medicines or foods for treating or preventing thrombosis. Development of such proteases is therefore desired.
Patent Document 1: Japanese Unexamined Patent Publication No. 1991-277279

### DISCLOSURE OF THE INVENTION

### PROBLEMS TO BE SOLVED BY THE INVENTION

An object of the present invention is to provide a protease that is stable in a wide pH range from acidic to alkaline, and that has excellent thrombolytic activity; a protease-producing microorganism that produces the above protease; and a process for producing the protease. Another object of the present invention is to provide a thrombolytic agent or food containing the protease, and a fermented food produced using the protease-producing microorganism.

### MEANS FOR SOLVING THE PROBLEMS

The present inventor conducted extensive research to achieve the above objects, and found that a filamentous fungus belonging to the genus *Fusarium* (*Fusarium* sp. strain BLB; FERM BP-10493), which was isolated from tempeh prepared using hibiscus leaves, produces a novel protease that is stable in a wide pH range from acidic to alkaline and that has excellent thrombolytic activity. The inventor also found that the protease can be used as a material for foods and medicines. Further, the inventors found that the microorganism is edible, and that a fermented food obtained by fermenting beans or grains using the microorganism contains the above-mentioned protease and is useful as a food for treating or preventing thrombosis, or a health food for other purposes. The present invention was accomplished by making improvements based on these findings.

The present invention provides the following protease, protease-producing microorganism, protease production process, thrombolytic agent, food, and fermented food:
Item 1. A protease having the following properties:
   (1) activity/substrate specificity: having fibrinolytic activity, and degrading activity on synthetic substrates H-D-Ile-Pro-Arg-pNA, H-D-Val-Leu-Lys-pNA, and Bz-L-Arg-pNA;
   (2) active pH and optimum pH: being active at least within a pH range of 6.5 to 11.5, and being optimally active at about pH 8.5 to about 9.5;
   (3) pH stability: being stable at least within a pH range of 2.5 to 11.5 under treatment conditions of 4°C and 20 hours;
   (4) active temperature and optimum temperature: being active at least within a temperature range of 30 to 50°C, and being optimally active at about 45 to about 50°C;
   (5) temperature stability: being stable at at least about 55°C under treatment conditions of pH 5 and 10 minutes;
   (6) molecular weight: having an estimated molecular weight of about 27000 on SDS-PAGE;
   (7) inhibitory properties: not being inhibited by 0.01 mg/ml SBTI but being inhibited by 1 mM PMSF and 0.1 mM DFP.
Item 2. The protease according to item 1, which is derived from a microorganism belonging to the genus *Fusarium.*
Item 3. The following protein (a) or (b):
   (a) a protein consisting of the amino acid sequence represented by SEQ ID NO: 1;
   (b) a protein consisting of an amino acid sequence derived from the amino acid sequence represented by SEQ ID NO: 1 by deletion, substitution or addition of one or more amino acids, the protein being a protease having the properties (1) and (7) shown in item 1.
Item 4. A gene encoding the protein according to item 3.
Item 5. A gene consisting of the following DNA (i) or (ii):
   (i) a DNA consisting of the nucleotide sequence represented by SEQ ID NO: 2;
   (ii) a DNA that hybridizes, under stringent conditions, with a DNA consisting of a nucleotide sequence complementary to the DNA consisting of the nucleotide sequence represented by SEQ ID NO: 2, and that encodes a protein that is a protease having the properties (1) and (7) shown in item 1.
Item 6. A gene encoding any one of the following proteins (a), (b), and (c):
   (a) a protein consisting of the amino acid sequence represented by SEQ ID NO: 1;
   (b) a protein consisting of an amino acid sequence derived from the amino acid sequence represented by SEQ ID NO: 1 by deletion, substitution or addition of one or more amino acids, the protein being a protease having the properties (1) and (7) shown in item 1.
   (c) a protein having at least 80% homology with the amino acid sequence represented by SEQ ID NO: 1, the protein being a protease having the properties (1) and (7) shown in item 1.
Item 7. A recombinant vector containing a gene according to any one of items 4 to 6.
Item 8. A transformant containing the recombinant vector according to item 7.
Item 9. A process for producing a protease, the process comprising culturing the protease-producing microorganism according to item 8 and recovering a protease from the culture medium.
Item 10. A protease-producing microorganism that belongs to the genus *Fusarium* and that produces the protease according to item 1.
Item 11. The protease-producing microorganism according to item 10, the microorganism being characterized by:
   (iii) having an ITS-5.8S rDNA consisting of the nucleotide sequence represented by SEQ ID NO: 3 or a nucleotide sequence having at least 98% homology therewith; or
   (iv) having a 28S rDNA consisting of the nucleotide sequence represented by SEQ ID NO: 4 or a nucleotide sequence having at least 98% homology therewith.
Item 12. The protease-producing microorganism according to item 10, which is *Fusarium* sp. strain BLB (FERM BP-10493).
Item 13. A process for producing a protease, the process comprising culturing a protease-producing microorganism according to any one of items 10 to 12 and recovering a protease from the culture medium.
Item 14. A thrombolytic agent containing the protease according to item 1 or the protein according to item 3.
Item 15. A method for treating or preventing thrombosis, comprising administering, to a thrombosis patient or a person who needs prophylactic treatment for thrombosis, the protease according to item 1 or the protein according to item 3, in an amount effective for treating or preventing thrombosis.
Item 16. Use of the protease according to item 1 or the protein according to item 3 for the manufacture of a thrombolytic agent.
Item 17. A food containing the protease according to item 1 or the protein according to item 3.
Item 18. A fermented food obtained by inoculating a food material with a protease-producing microorganism according to any one of items 10 to 12, and fermenting the food material.

### EFFECTS OF THE INVENTION

The protease of the present invention has excellent thrombolytic activity and is stable in a wide pH range from acidic to alkaline, and therefore finds wide applications in industrial fields, especially in the fields of foods, medicines, etc.

Further, fermented foods produced by using the protease-producing microorganism of the present invention exhibit useful physiological activity based on the activity of the protease, and are therefore valuable health foods.

### BRIEF DESCRIPTION OF THE DRAWINGS

[Fig. 1] Figure 1 shows the active pH and optimum pH of the protease of the present invention (derived from *Fusarium* sp. BLB).
[Fig. 2] Figure 2 shows the pH stability of the protease of the present invention (derived from *Fusarium* sp. BLB).
[Fig. 3] Figure 3 shows the active temperature and optimum temperature of the protease of the present invention (derived from *Fusarium* sp. BLB).
[Fig. 4] Figure 4 shows the temperature stability of the protease of the present invention (derived from *Fusarium* sp. BLB).

### BEST MODE FOR CARRYING OUT THE INVENTION

The present invention is described below in detail.

### 1. Protease

The enzymological properties of the protease of the present invention are described below.

### Protease-Activity-Measuring Method: Fibrin Plate Method

A bovine plasma-derived fibrinogen (Sigma) is dissolved in 0.1 M phosphate buffer (pH 7.2) to a concentration of 0.5 wt.%. The insoluble matter is filtered off using filter paper (Toyo Roshi, No. 2). The solution is dispensed in 20 ml aliquots into square petri dishes (No. 2; 144 x 104 x 16 mm), and 100 µl of 50 U/ml thrombin solution is added to each petri dish while stirring. After formation and coagulation of fibrin, pre-incubation is carried out at 37°C for 30 minutes. After dropping 30 µl of sample (protease solution) onto each fibrin plate (artificial thrombus), the fibrin plates are allowed to stand at 37°C for 4 hours, and the lysis area (major axis x minor axis) is measured. The area can be converted into the international units of urokinase by comparison with the lysis area measured in the same manner using urokinase.

### Protease-Activity-Measuring Method: Casein Method

A 0.5 ml quantity of sample (protease solution) is added to 1.5 ml of Hammarsten casein solution, prepared using 100 mM boric acid buffer (pH 10) so as to have a final concentration of 1 wt.%, and a reaction is carried out at 37°C for 10 minutes. The reaction is terminated by adding 2 ml of 0.44 M trichloroacetic acid solution. After allowing the reaction mixture to stand for 20 minutes, the precipitate is filtered off. Five milliliters of 0.44 M aqueous sodium carbonate solution and 1 ml of phenol reagent (containing, per 100 ml, 9.1 g of sodium tungstate dihydrate, 2.3 g of sodium molybdate dihydrate, 4.5 ml of phosphoric acid, 9.1 ml of hydrochloric acid, and 13.6 g of lithium sulfate) are added in that order. After allowing the resulting mixture to stand for 20 minutes, the absorbance at an absorption wavelength of 660 nm is measured. In the above measurement, the amount of enzyme that liberates, per minute, an acid-soluble protein hydrolysate corresponding to 1 µg of tyrosine is defined as 1 unit.

### Protease-Activity-Measuring Method: Synthetic Substrate Method

Five microliters of 50 mM synthetic substrate and 455 µl of sample (protease solution) are added to 500 µl of each of various 200 mM buffer solutions. A reaction is carried out at 37°C for 10 minutes, and the absorbance at an absorption wavelength of 405 nm is measured. In the above measurement, the amount of enzyme that liberates 1 nmol of p-nitroaniline per minute is defined as 1 unit.

### (1) Activity and Substrate Specificity

The protease of the present invention has strong fibrinolytic activity. Table 1 shows the degrading activity (relative activity (%)) on various synthetic substrates, based on the degrading activity on a synthetic substrate H-D-Ile-Pro-Arg-pNA being taken as 100. The protease has degrading activity on synthetic substrates H-D-Ile-Pro-Arg-pNA, H-D-Val-Leu-Lys-pNA, and Bz-L-Arg-pNA; and in particular, it has strong degrading activity on H-D-Ile-Pro-Arg-pNA and H-D-Val-Leu-Lys-pNA. However, it has no degrading activity on synthetic substrates Suc-Ala-Ala-Pro-Leu-pNA, Suc-Ala-Ala-Pro-Phe-pNA, or Gle-Phe-pNA.

**[Table 1]**

| Substrate | Relative Activity (%) |
|---|---|
| H-D-Ile-Pro-Arg-pNA | 100 |
| H-D-Val-Leu-Lys-pNA | 21 |
| Bz-L-Arg-pNA | 2.8 |
| Suc-Ala-Ala-Pro-Leu-pNA | 0 |
| Suc-Ala-Ala-Pro-Phe-pNA | 0 |
| Gle-Phe-pNA | 0 |

### (2) Active pH and Optimum pH

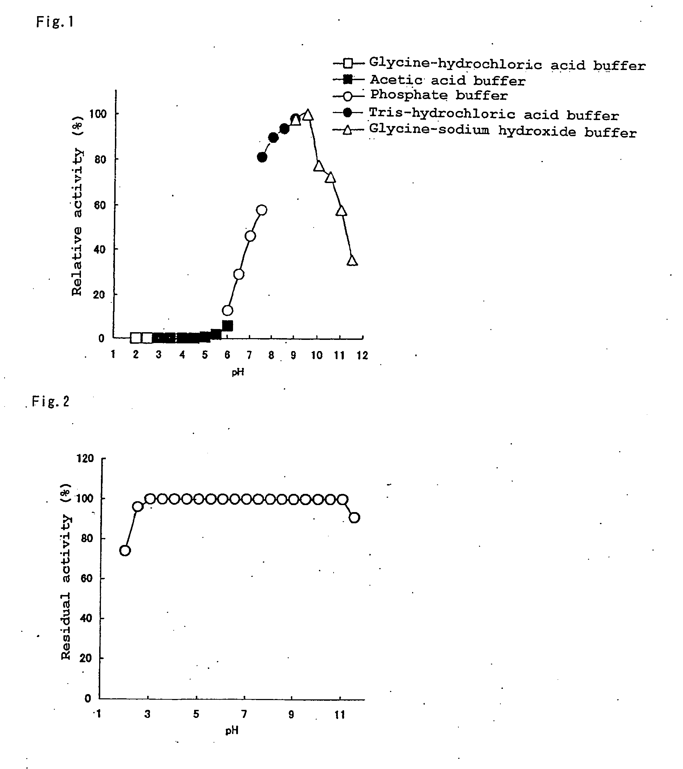

Figure 1 shows the results of measuring the degrading activity on Bz-L-Arg-pNA in buffers (glycine-hydrochloric acid buffer at pH 2 to 3, acetic acid buffer at pH 3.5 to 6, phosphate buffer at pH 6 to 8, tris-hydrochloric acid buffer at pH 8 to 9, and glycine-sodium hydroxide buffer at a pH 9 to 12). As shown in Fig. 1, the protease is active at least within a pH range of 6.5 to 11.5, and is optimally active at a pH of about 8.5 to about 9.5. As used herein, "active" means showing an activity of at least 30% relative to the activity at the optimum pH (pH 9.5) taken as 100%.

### (3) pH Stability

The protease was added to 50 mM buffers (glycine-hydrochloric acid buffer at pH 2 to 3, acetic acid buffer at pH 3.5 to 6, phosphate buffer at pH 6 to 8, tris-hydrochloric acid buffer at pH 8 to 9, and glycine-sodium hydroxide buffer at pH 9 to 12), and allowed to stand at 4°C for 20 hours. The residual activity of the protease thus treated was measured using a synthetic substrate Bz-L-Arg-pNA in glycine-sodium hydroxide buffer (pH 9.5) (see Fig. 2). As shown in Fig. 2, the protease is stable at least within a pH range of 2.5 to 11.5 under treatment conditions of 4°C for 20 hours. As used herein, "stable" means showing a residual activity of at least 90%.

### (4) Active Temperature and Optimum Temperature

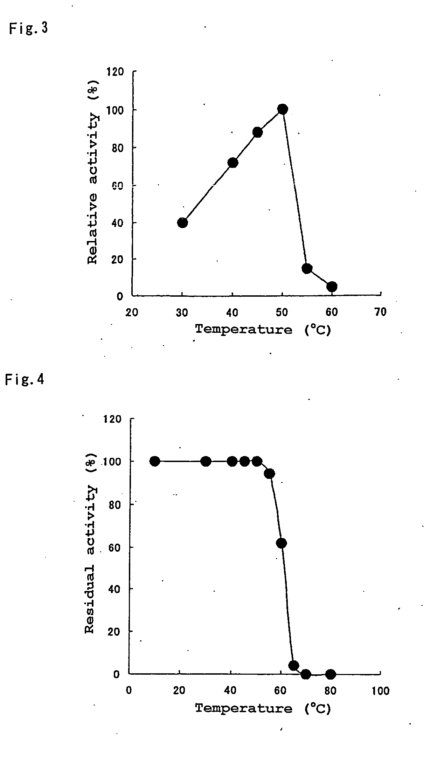

Figure 3 shows the results of measuring the degrading activity on a synthetic substrate Bz-L-Arg-pNA in 100 mM acetic acid buffer (pH 5.0) at 30 to 60°C. As shown in Fig. 3, the protease is active at least within a temperature range of 30 to 50°C, and is optimally active at about 45 to about 50°C. As used herein, "active" means showing an activity of at least 30% relative to the activity at the optimum temperature (50°C) taken as 100%.

### (5) Temperature Stability

The protease was added to 50 mM acetic acid buffer (pH 5.0), and allowed to stand at 20 to 80°C for 10 minutes. The residual activity of the protease thus treated was measured using a synthetic substrate Bz-L-Arg-pNA in glycine-sodium hydroxide buffer (pH 9.5) (see Fig. 4). As shown in Fig. 4, the protease is stable at at least about 55°C under treatment conditions of pH 5 and 10 minutes. As used herein, "stable" means showing a residual activity of at least 90%.

### (6) Molecular Weight

The estimated molecular weight on SDS-PAGE (the method of Laemmli) is about 27000.

### (7) Inhibitory Properties

After allowing the protease to stand in 50 mM acetic acid buffer (pH 5) in the presence of each of various protease inhibitors at 37°C for 1 hour, the protease activity was measured using a synthetic substrate Bz-L-Arg-pNA. Table 2 shows the relative activity (%) calculated with the activity in the absence of inhibitors being taken as 100. Table 2 reveals that the protease is inhibited by 1 mM PMSF and 0.1 mM DFP, but is not inhibited by 0.01 mg/ml SBTI, or the other protease inhibitors shown in Table 2.

**[Table 2]**

| Inhibitor | Concentration | Relative Activity (%) |
|---|---|---|
| SBTI | 0.01 mg/ml | 100 |
| SSI | 0.01 mg/ml | 100 |
| ε-ACA | 1 mM | 100 |
| PMSF | 1 mM | 59 |
| TPCK | 0.1 mM | 97 |
| DFP | 0.1 mM | 21 |
| 2,2'-Bipyridyl | 1 mM | 97 |
| Phenanthroline | 1 mM | 100 |
| EDTA | 1 mM | 100 |
| E-64 | 0.1 mM | 97 |
| Chimostatin | 0.01 mg/ml | 100 |
| Pepstatin | 0.1 mM | 100 |
| SPI | 0.1 mM | 100 |

As used herein, the abbreviations indicating protease inhibitors are as follows. SBTI: soybean trypsin inhibitor; SSI: *Streptomyces subtilisin* inhibitor; ε-ACA: ε-aminocaproic acid; PMSF: phenylmethylsulfonyl fluoride; TPCK: N-tosyl-L-phenylalanyl chloromethyl ketone; DFP: diisopropylfluorophosphate; EDTA: ethylenediaminetetraacetic acid; E-64: t-epoxysuccinyl-L-leucylamide(4-guanidino)butane, SPI: *Streptomyces* pepsin inhibitor.

In view of the above enzymological properties, although the protease of the present invention can be regarded as a trypsin-type serine protease, it is a novel protease that is clearly different from known serine proteases, in view of the properties of being uninhibited by SBTI, substrate specificity, and stability in a wide pH range.

Since the protease of the present invention is stable in a wide pH range, it is widely applicable in various fields. For example, it can be used not only as a thrombolytic agent or food as described hereinafter, but also for breaking down hardly degradable proteins, softening meat, producing amino acids, producing physiologically active peptides that are usable in medicines or foods, producing bread, producing fermented foods (e.g., cheese), and other purposes.

The present invention provides, from the viewpoint of amino acid sequences, the following proteins (a), (b), and (c):
(a) a protein consisting of the amino acid sequence represented by SEQ ID NO: 1;
(b) a protein consisting of an amino acid sequence derived from the amino acid sequence represented by SEQ ID NO: 1 by deletion, substitution or addition of one or more amino acids, the protein being a protease having the above-mentioned properties (1) and (7); and
(c) a protein having at least 80% homology with the amino acid sequence represented by SEQ ID NO: 1, the protein being a protease having the above-mentioned properties (1) and (7).

The amino acid sequence represented by SEQ ID NO: 1 corresponds to an amino acid sequence encoded by an open reading frame (ORF) of the nucleotide sequence represented by SEQ ID NO: 2.

In the above protein (b), the number of the "one or more amino acids" is not limited, and is, for example, 1 to 50, preferably 1 to 25, more preferably 1 to 12, even more preferably 1 to 9, and still more preferably 1 to 5.

Techniques for substituting, deleting, or adding one or more amino acids in a specific amino acid sequence are known.

The above protein (c) has at least 80% homology, preferably at least 90% homology, and more preferably 95% homology, with the amino acid sequence represented by SEQ ID NO: 1.

The homology of amino acid sequences can be calculated using analysis tools that are commercially available or available through an electronic communications network (the Internet). Specifically, the homology can be calculated using an analysis software BLAST (J. Mol. Biol., 215, 403, 1990).

The proteins (b) and (c) preferably have such protease activity that satisfies, in addition to the properties (1) and (7), at least one of the properties (2) to (5), and more preferably all the properties (2) to (5).

### 2. Protease-Producing Microorganism

The present invention provides a microorganism belonging to the genus *Fusarium,* as a microorganism that produces the above protease (protease-producing microorganism). The protease-producing microorganism is not limited, as long as it belongs to the genus *Fusarium* and is capable of producing a protease having the above-mentioned properties. Examples of such microorganisms include *Fusarium* sp. strain BLB isolated from tempeh prepared using hibiscus leaves. *Fusarium* sp. strain BLB is able to produce a protein consisting of the amino acid sequence represented by SEQ ID NO: 1. It has been confirmed that *Fusarium* sp. strain BLB does not have any mycotoxin-producing ability. The following are the mycological properties and genetic properties of *Fusarium* sp. strain BLB.

### (i) Mycological Properties

The strain exhibits the following properties when it is inoculated into plates of a Bacto Potato Dextrose Agar (Becton Dickinson and Co.), Bacto Oatmeal Agar (Becton Dickinson and Co.), or Bacto Malt Extract-containing agar medium (containing 2 wt.% Bacto Malt Extract (Becton Dickinson and Co.) + 1.5 wt.% agar), and incubated at 25°C for a maximum period of six weeks.

### (a) Growth

In all of the plates at 25°C, the strain grows rapidly and covers the entire surfaces of the plates with a diameter of 85 mm, within ten days of incubation.

### (b) Mycelium

The mycelium is velutinous to floccose. The surface is white from the initial stage, and no coloring on the back is found. No change on the colony surface due to the adhesion of conidia is observed.

### (c) Soluble Pigment

No production of soluble pigments is found.

### (d) Conidia

Microconidia and macroconidia are formed. The microconidia are phialidic, and have a conidiophore structure similar to that of the genus *Acremonium.* The conidiophores are formed almost singly, and many of the stipes formed are relatively long. The microconidia consist of one or two cells, are viscous, slimy at the tip portion of the strips, fusiform, and have smooth surfaces. The macroconidia are formed at the base portion of the aerial mycelia, consist of two to four cells, are luniform, and have smooth surfaces and foot cells. Many of the macroconidia formed have a medium thickness, and a medium length. (ii) Genetic Characteristics

SEQ ID NO: 3 in the sequence listing represents the nucleotide sequence of the ITS-5.8S rDNA region (internal transcription spacer region and 5.8S ribosomal RNA gene) (hereinafter referred to as ITS-5.8SrDNA) contained in the chromosomal DNA of *Fusarium* sp. strain BLB. SEQ ID NO: 4 of the sequence listing represents the nucleotide sequence of the 28S ribosomal RNA gene (hereinafter 28S rDNA) contained in the chromosomal DNA of *Fusarium* sp. strain BLB. The nucleotide sequences of ITS-5.8S rDNA and 28S rDNA were determined by extracting the genomic DNA from *Fusarium* sp. strain BLB, carrying out PCR using the genomic DNA as a template to amplify the ITS-5.8S rDNA and 28S rDNA regions, and determining the full-length nucleotide sequences by a standard method. The PCR amplification of the ITS-5.8S rDNA was performed using primers ITS5 and ITS4 (White, T. J., T. Bruns, S. Lee, and J. W. Tayer. (1990), Amplification and Direct Sequencing of Fungal Ribosomal RNA Gene for Phylogenetics, in Innis, M. A., Gelfand, D. H., Sninsky, J.J., and White, T. J. (eds.), PCR Protocols, A Guide to Methods and Applications, Academic Press, Inc., New York, pp. 315-322); and the PCR amplification of 28S rDNA was performed using the primers NL1 and NL2 (O'Donnell, K. (1993), Fusarium and Its Near Relatives, in Reynolds, D. R. and Tayor, and J. W. (Eds.), The Fungal Holomorph: Mitotic, Meiotic and Pleomorphic Speciation in Fungal Systematics, CAB International Wallingford, UK, pp. 225-233).

A BLAST search of the GenBank was carried out using the nucleotide sequences of ITS-5.8S rDNA and 28S rDNA of *Fusarium* sp. strain BLB as queries, and it was confirmed that *Fusarium* sp. strain BLB is a strain belonging to the genus *Fusarium* and that its species is unknown.

*Fusarium* sp. strain BLB was deposited on January 20, 2005 under accession number FERM P-20370 at the Patent Organism Depositary, National Institute of Advanced Industrial Science and Technology (Tsukuba Central 6, 1-1, Higashi 1-Chome, Tsukuba-shi, Ibaraki-ken 305-8566 Japan). The strain has been transferred to an international depository, and its accession number is FERM BP-10493.

Other specific examples of microorganisms that produce the protease, other than *Fusarium* sp. strain BLB, include filamentous fungi having an ITS-5.8S rDNA consisting of the nucleotide sequence represented by SEQ ID NO: 3 or a nucleotide sequence having at least 98% homology therewith; filamentous fungi having a 28S rDNA consisting of the nucleotide sequence represented by SEQ ID NO: 4 or a nucleotide sequence having at least 98% homology therewith.

### 3. Protease Production Process

The protease production process of the present invention can be carried out by culturing the protease-producing microorganism mentioned above, and recovering a protease from the culture medium.

The medium used for the production process of the present invention is not limited as long as it is a suitable medium in which the microorganism can readily grow and produce the protease. A synthetic or natural medium containing a suitable carbon source, nitrogen source, inorganic salt, and other nutrients can be used. Examples of the carbon source of the medium include glucose, sucrose, fructose, maltose, glycerol, dextrin, oligosaccharides, starch, molasses, corn steep liquor, malt extract, organic acids, etc. Examples of nitrogen sources include organic nitrogen sources, such as corn steep liquor, yeast extract, various peptones, soybean flour, meat extract, bran extract, casein, amino acids, urea, etc.; inorganic nitrogen sources, such as nitrates, ammonium salts, etc.; and the like. Examples of inorganic salts include sodium salts, potassium salts, magnesium salts, iron salts, other metal salts, etc. Examples of other nutrients include vitamins, amino acids, nucleic acids, etc.

The culture may be solid culture or liquid culture, and can be carried out according to a general culture method for microorganisms. Liquid culture is preferable. The liquid culture can be carried out by aerated agitating culture, shaking culture, or the like. When liquid culture is employed, the culture method may be batch culture, feeding culture, or continuous culture. Culture conditions (temperature, pH, etc.) can be suitably selected according to the growth characteristics of the protease-producing microorganism to be cultured. The culture temperature is, for example, 20 to 35°C, and preferably 25 to 30°C. The culture pH is, for example, 4 to 8, and preferably 5 to 7. The culture period varies depending on the culture method, the kind and amount of medium, the temperature and pH conditions, etc., and cannot be generally defined, but may be usually 24 to 120 hours, and preferably about 60 to about 90 hours.

A protease is accumulated in the thus cultured cells and the culture supernatant, and can be eluted from the cells by a standard method. Specifically, for example, the culture medium itself, or the cells separated by centrifugation, filtration or like operation, can be subjected to mechanical disruption treatment, such as ultrasonication, treatment with a French press or high-pressure homogenizer, or the like; treatment with cyclohexane, toluene, ethyl acetate, or the like; or lysis treatment with lysozyme; to thereby elute the protease from the cells. If necessary, the protease eluate thus obtained and the protease-containing supernatant can be purified to a desired purity and concentration. The protease can be purified, for example, by a suitable combination of one or more treatments selected from solvent extraction, resin treatments (e.g., ion exchange, adsorption, molecular sieving, etc.), membrane treatments (e.g., membrane filtration, ultrafiltration, microfiltration, reverse osmosis, etc.), activated carbon treatment, supercritical fluid extraction treatment, distillation treatment, crystallization, and other treatments, which can be carried out in an arbitrary order, thereby recovering a fraction with protease activity.

The protease of the present invention can be produced by, as well as the above process, culturing a transformant into which a gene encoding the amino acid sequence of the protease has been introduced, as described hereinafter.

### 4. Gene Encoding the Protease, Recombinant Vector, and Transformant Gene

The present invention further provides a gene encoding the above protease.

Specific examples of the gene include a gene encoding any one of the proteins (a), (b), and (c). As described above, techniques for substituting, deleting, or adding one or more amino acids in a specific amino acid sequence are known, and a gene encoding the protein (b) mentioned above can be produced by a known method using a commercially available kit or the like.

Other specific embodiments of genes encoding the above protease include polynucleotides consisting of the following DNA (i) or (ii):
(i) a DNA consisting of the nucleotide sequence represented by SEQ ID NO: 2;
(ii) a DNA that hybridizes, under stringent conditions, with a DNA consisting of a nucleotide sequence complementary to the nucleotide sequence represented by SEQ ID NO: 2, and that has degrading activity on fibrin, H-D-Ile-Pro-Arg-pNA, H-D-Val-Leu-Lys-pNA, and Bz-L-Arg-pNA, and that encodes a protein whose degrading activity on Bz-L-Arg-pNA is not inhibited by 0.01 mg/ml SBTI.

With respect to the DNA (ii), the stringent conditions are, for example, such that the hybridization is carried out at 65°C in a 5x SSC solution (1x SSC solution is composed of 150 mM sodium chloride and 15 mM sodium citrate), followed by washing with a 0.5x SSC solution containing 0.1% of SDS. Each step of hybridization under stringent conditions can be carried out by known methods, such as the method described in "Molecular Cloning (Third Edition)" (J. Sambrook & D. W. Russell, Cold Spring Harbor Laboratory Press, 2001), etc. Usually, the stringency increases as the temperature increases and as the salt concentration decreases.

The DNA to be hybridized under stringent conditions usually has more than a certain level of homology with the nucleotide sequence of the DNA used as a probe. The homology is, for example, at least 70%, preferably at least 80%, and more preferably at least 90%, and still more preferably at least 95%. The homology of nucleotide sequences can be calculated using analysis tools that are commercially available or available through an electronic communications network (the Internet).

Specifically, the homology can be calculated using an analysis software BLAST (J. Mol. Biol., 215, 403, 1990).

The gene can be obtained by RT-PCR using, as a template, a total mRNA prepared from the above-mentioned protease-producing microorganism by a standard method, and primers designed to amplify the full length of the gene. The gene can also be obtained by PCR using, as a template, a cDNA library constructed from the protease-producing microorganism, and primers designed to amplify the full length of the gene.

The PCR amplification of the gene can be carried out by repeating a heating-cooling cycle in a reaction mixture containing a cDNA used as a template, PCR buffer, primer pair (forward primer and reverse primer), dNTP mixture (deoxynucleotide-triphosphate mixture), and DNA polymerase. The forward primer and reverse primer are designed based on about 10 to about 40 bp nucleotide sequence located at or near the 5' end or at or near the 3' end of the gene, and are synthesized by a standard method. Specific examples of the primer pair used in the PCR include a primer pair of TempeRTForward1 primer (5'-CCTTCGCCTGTTCTTCATCAT-3') and TempeRTRevese1 primer (5'-AGTACCTAAGCCAAAATATGC-3'). The PCR buffer can be suitably selected according to the DNA polymerase and the like used in the PCR, and may be a commercial product. The dNTP mixture and DNA polymerase may also be commercial products. The PCR reaction can be carried out according to a conventional procedure or the DNA polymerase protocol, in which the reaction temperature, reaction time, reaction cycle, reaction mixture composition, etc., can be suitably modified. The PCR conditions may be, for example, such that a reaction cycle consisting of denaturation (98°C, 20 sec.), annealing (55°C, 20 sec.), and extension (68°C, 60 sec.) is performed 30 times.

### Recombinant Vector

The above-mentioned gene is used by introducing it into a suitable vector. Vectors that can be used in the present invention include autonomously replicating vectors (e.g., plasmids), and vectors that, when introduced into host cells, are integrated into the genomes of the host cells and replicate along with the host chromosomes. Specifically, such vectors include vectors derived from bacterial plasmids, bacteriophages, transposons, viruses (e.g., baculoviruses, papovaviruses, SV40, vaccinia viruses, adenoviruses, fowlpox viruses, pseudorabies viruses, retroviruses, etc.); plasmids and vectors derived from genetic elements of bacteriophages (e.g., cosmids, phagemids, etc.).

Such vectors are preferably expression vectors. In the expression vectors, elements of the gene that are necessary for transcription (e.g., promoter and the like) are functionally linked.

Recombinant vectors containing the gene comprise elements such as the sequence of the gene, sequences carrying information for replication and control (e.g., promoter, ribosome binding site, terminator, signal sequence, enhancer, etc.), a selection marker gene sequence, etc., and are produced by combining these elements using a known method.

The above gene can be inserted into a vector DNA using a known method. For example, the DNA and vector DNA can be cleaved at specific sites using suitable restriction enzymes, and mixed for ligation with ligases. A recombinant vector can also be obtained by ligating a suitable linker to the gene, and inserting the gene with the linker into a multicloning site of a vector that is suitable for the purpose.

### Transformant

A transformant into which the above-mentioned gene has been introduced can be obtained by introducing, by a known method, a recombinant vector in which the above gene has been incorporated, into known host cells such as *Escherichia coil, Bacillus* bacteria, and like bacteria; yeasts; insect cells; animal cells; etc. The method for introducing the gene is not limited, and is preferably integration into chromosomes. The method for introducing the recombinant vector into a host cell, can be suitably selected from known methods depending on the type of host cell. Specifically, the recombinant vector can be introduced into a host cell by, for example, calcium phosphate transfection, DEAE-dextran-mediated transfection, microinjection, cationic lipid-mediated transfection, electroporation, etc.

The protease of the present invention can be produced by culturing the transformant into which the gene has been introduced, and recovering the protease of the present invention from the culture medium.

The culturing can be carried out by subculture or batch culture using a medium suitable for the host. The culturing is performed until a suitable amount of protease of the present invention has been obtained, using, as an index, the amount of protease produced inside and outside the transformant.

The protease can be recovered by the method described in "2. Protease Production Process" above.

### 5. Thrombolytic Agent and Food

The above-mentioned protease has excellent thrombolytic activity, and is useful for the treatment and prevention of thrombosis when it is used as an active ingredient of a thrombolytic agent. For example, the protease, either by itself or enclosed in microcapsules, can be directly injected intravenously as a thrombolytic agent. The above protease can be formulated into tablets, powder, granules, capsules, liquid, or the like by a known method, and can be orally administered as a thrombolytic agent.

The dose of the thrombolytic agent varies depending on the sex and age of the subject to whom the agent is administered, the severity of the symptom of thrombosis, the forms and administration method of the agent, etc. Usually, for example, the dose is selected so that 0.001 to 20 g/day, and preferably 0.01 to 10 g/day, of protease is administered.

It is desirable that the thrombolytic agent be formulated into dosage units each containing the above-mentioned amount of protease.

The protease not only has thrombolytic activity but also stability in a wide pH range, and therefore can be added to various forms of foods. Foods containing the protease are useful as foods for treating or preventing thrombosis, and are also useful as readily absorbable foods, protein-enriched foods, etc.

The proportion of the protease in the above protease-containing food can be suitably selected depending on the form of food and other factors, and is usually about 0.0001 to 20 wt.%, and preferably 0.001 to 10 wt.%.

The daily intake of the food varies depending on the form of food, the sex and age of the person who ingests the food, etc., and is, for example, 0.001 to 20 g, and preferably 0.01 to 10 g, calculated as the daily intake of protease.

### 6. Fermented Food

Since the above-mentioned protease-producing microorganism has no safety problems and is harmless to the human body, it can be eaten as it is and can be used for producing fermented foods. That is, the present invention further provides a fermented food obtained by inoculating a food material with the protease-producing microorganism and fermenting the food material.

Examples of food materials that can be used for producing the fermented food include beans such as soybeans, peanuts, *adzuki* beans, broad beans, etc.; grains such as rice, wheat, etc.; coconut; *okara* (residue left after making tofu); and the like. The fermented food of the present invention is preferably one prepared using beans as a food material.

The fermented food can be produced by adding water to a food material to give a water content of 30 to 70 wt.%, performing sterilization if necessary, and then inoculating the food material with the protease-producing microorganism, followed by incubation at 20 to 35°C for 24 to 72 hours. If necessary, a substance that promotes the growth of the protease-producing microorganism (e.g., starch or the like) can be added to the food material.

Since the fermented food contains the protease of the present invention, the food exhibits various physiological effects such as thrombolytic effects, based on the activity of the protease, and thus is useful as a health food. Further, as compared with tempeh prepared using conventional tempeh fungus, fermented soybean foods prepared using the protease-producing microorganism have a different flavor and a new palatability, and are superior in that they exhibit excellent physiological effects based on the activity of protease.

### EXAMPLES

The present invention is described below in detail with reference to Examples, but are not limited thereto.

### Example 1 Production of Protease

One platinum loop of *Fusarium* sp. strain BLB (FERM BP-10493) isolated from tempeh prepared using hibiscus leaves was inoculated into 30 ml of liquid medium (containing 2 wt.% of defatted soybean powder, 2 wt.% of glucose, 0.5 % of polypeptone, 0.2 wt.% of yeast extract, 0.1 wt.% of KH₂PO₄, and 0.05 wt.% of MgSO₄), and shaking culture was carried out at 28°C for 72 hours to give a preculture medium. Subsequently, 15 ml of preculture medium was inoculated into 1.5 1 of liquid medium (containing 4 wt.% of defatted soybean powder, 3 wt.% of glucose, 0.2 wt.% of yeast extract, 0.1 wt.% of KH₂PO₄, 0.1 wt.% of K₂HPO₄, 0.05 wt.% of MgSO₄ and 0.03 wt.% of silicon), and cultured in a jar fermenter with an aeration of 0.5 VVM at 28°C for 72 hours.

The obtained culture medium was subjected to solid-liquid separation using a filter press. The protease activity of the obtained culture supernatant was measured by the above-mentioned casein method and found to be 68.2 units/ml. The protease activity of the obtained culture supernatant was measured by the above-mentioned fibrin plate method and found to be 1500 IU/ml.

### Example 2 Purification of Protease

Ammonium sulfate was added to 3500 ml of culture supernatant obtained in Example 1 to achieve 70% saturation and thereby salt out the protein. Centrifugation was performed, and the obtained precipitate was dissolved in 100 ml of 20 mM acetic acid buffer (pH 5.0) and dialyzed against the buffer to remove the salt. Further, 350 ml of the dialysis residue was passed through a CM-TOYOPEARL column (4.5 x 30 cm, TOSOH CORP.) equilibrated with the same buffer to adsorb the protein onto the column, and the adsorbed protein was eluted by the linear density gradient method using the same buffer at a NaCl concentration of up to 0.5 M. Then, 190 ml of fraction having caseinolytic and fibrinolytic activity was recovered, and ammonium sulfate was again added to achieve 70% saturation and thereby salt out the protein. Centrifugation was performed, and the obtained precipitate was dissolved in 3 ml of the same buffer containing 0.2 M NaCl, and the solution was subjected to gel filtration on a Superdex 75 column (Amersham Bioscience) to recover a fraction having caseinolytic and fibrinolytic activity.

It was confirmed that, in the fraction (final purified product) thus obtained, a protease having the following properties had been purified: (1) The activity and substrate specificity of the final purified product were as shown in Table 1 above. The protease activity of the final purified product was 634 U/mg as measured by the casein method. (2) The active pH and optimum pH of the final purified product were as shown in Fig. 1. (3) The pH stability of the final purified product was as shown in Fig. 2. (4) The active temperature and optimum temperature of the final purified product were as shown in Fig. 3. (5) The temperature stability of the final purified product was as shown in Fig. 4. (6) SDS-polyacrylamide gel electrophoresis of the final purified product revealed a single band (estimated molecular weight: 27000). (7) The influences of inhibitors on the final purified product were as shown in Table 2 above.

An acute toxicity test on mice demonstrated the safety of the final purified product thus obtained. The final purified product was also confirmed negative for mutation induction.

### Example 3 Identification of Amino Acid Sequence of the Protease and Nucleotide Sequence Encoding the Protease

### Identification of Genomic DNA Sequence Encoding the Protease Obtained in Example 2

The N-terminal amino acid sequence of the protein obtained in Example 2 was analyzed. The analysis revealed that the N-terminal amino acid sequence of the protein obtained in Example 2 has a homology with trypsin derived from *Fusarium oxysporum, Phaeosphaeria nodorum* SNP1, and *Verticillium dahliae.* Thus, considering the above information, primer pair A (5'-GGCGACTTTCCCTTCATCGTGAGCAT-3' and 5'-TCACCCTGGCAAGAGTCCTTGCCACC-3') was designed. A PCR reaction was carried out using primer pair A and the genomic DNA of *Fusarium* sp. strain BLB (FERM BP-10493) as a template. LA Taq polymerase (TaKaRa) was used in the PCR reaction. The genomic DNA was extracted from *Fusarium* sp. strain BLB using ISOPLANT (NIPPON GENE). This amplified an about 600 bp DNA fragment.

Subsequently, the amplified fragment was sequenced to design new primer pair B (5'-ACCATTCCCATTGTCTCTCGCGCCACTT-3' and 5'-GGCGTTAAGAAGGGTACCACCGCACCAA-3'). Separately, the genomic DNA of *Fusarium* sp. strain BLB was subjected to SalI digestion, and then self-ligated. Using the self-ligated DNA as a template and primer pair B, an inverse PCR reaction was carried out. This amplified an about 2.5 Kbp DNA fragment.

The amplified fragment was sequenced to design new primer pair C (5'-CTTGCCAGGGTGACAGCGGTGGCCC-3' and 5'-CAAGGATCAGCATCCCGATGAGGAAAGT-3'). Separately, the genomic DNA of *Fusarium* sp. strain BLB was subjected to SacI digestion, and then self-ligated. Using the self-ligated DNA as a template and primer pair C, an inverse PCR reaction was carried out. As a result, an about 4 Kbp DNA fragment was amplified. The amplified fragment was sequenced, and the 1740 bp genomic nucleotide sequence (SEQ ID NO: 5) encoding the protease of the present invention was elucidated. A reagent manufactured by TaKaRa was used for the genetic manipulation described above. A Big Dye Terminator v3.1 Cycle Sequencing Kit (Applied Biosystems) was used for the DNA sequencing.

### Identification of cDNA Sequence Encoding the Protease Obtained in Example 2, and Amino Acid Sequence of the Protease

Since the genomic nucleotide sequence (SEQ ID NO: 5) identified above contains an intron, identification of the cDNA nucleotide sequence is necessary for specifying the region encoding the protease obtained in Example 2. Therefore, the cDNA sequence encoding the protease obtained in Example 2, and the amino acid sequence of the protease, were identified by the following method.

First, the total RNA was obtained using a culture medium of *Fusarium* sp. strain and a FastRNA Pro Kit (BIO 101). Then, a cDNA library was constructed from the obtained total RNA using a SuperScriptIII First Strand System (Invitrogen) and an oligo dT primer. Separately, a TempeRTForward1 primer (5'-CCTTCGCCTGTTCTTCATCAT-3') and a TempeRTRevese1 primer (5'-AGTACCTAAGCCAAAATATGC-3') were prepared. The target cDNA was amplified by PCR using the primer pair, LA Taq (TaKaRa), and the cDNA library obtained above. The PCR reaction was carried out by performing 30 cycles each consisting of 98°C for 20 sec., 55°C for 20 sec., and 68°C for 60 sec. Sequencing of the amplified DNA fragment of about 800 bp elucidated the cDNA sequence (SEQ ID NO: 2) encoding the protease obtained in Example 2, and the amino acid sequence (SEQ ID NO: 1) of the protease obtained in Example 2.

It was demonstrated that the amino acid sequence (SEQ ID NO: 1) of the protease obtained in Example 2 has 76% homology with trypsin derived from *Fusarium oxysporum*, and has 62% homology with trypsin derived from *Phaeosphaeria nodorum* SNP1. Example 4 Production of Fermented Food

One kilogram of soybeans was soaked overnight in 3 1 of 1.0 wt.% lactic acid solution and peeled. The peeled soybeans were then soaked in 0.1 wt.% lactic acid solution and boiled for 30 minutes. Subsequently, 20 g of starch was admixed with the cooked soybeans, and the beans were inoculated with 3 ml of preculture medium of *Fusarium* sp. strain BLB, and incubated at 28°C for 48 hours to produce fermented soybean food (tempeh). It was confirmed that the fermented soybean food (tempeh) thus obtained had a flavor different from tempeh prepared using the conventional tempeh fungus (*Rhizopus*), and has a new palatability.

One gram of fermented soybean food thus obtained was added to 4 ml of physiological salt solution, followed by stirring at 28°C for 3 hours, and centrifugation was performed to obtain the supernatant. The protease activity of the supernatant was measured by the fibrin plate method. For comparison, conventional tempeh fungus *(Rhizopus)* was used in place of *Fusarium* sp. strain BLB to prepare a fermented soybean food (tempeh) using the same method as above, and the protease activity was measured in the same manner. As a result, the lysis area was 70 mm² with respect to the tempeh produced using conventional tempeh fungus, whereas it was 150 mm² with respect to the fermented soybean food produced using *Fusarium* sp. strain BLB.

The above results demonstrate that the use of *Fusarium* sp. strain BLB in the production of a fermented soybean food (tempeh) provide a fermented food having a higher thrombolytic activity than that of a tempeh produced using the conventional tempeh fungus.

The safety of the fermented soybean food thus obtained was confirmed by an acute toxicity test on mice.

## Claims

1. A protease having the following properties:
(1) activity/substrate specificity: having fibrinolytic activity, and degrading activity on synthetic substrates H-D-Ile-Pro-Arg-pNA, H-D-Val-Leu-Lys-pNA, and Bz-L-Arg-pNA;
(2) active pH and optimum pH: being active at least within a pH range of 6.5 to 11.5, and being optimally active at about pH 8.5 to about 9.5;
(3) pH stability: being stable at least within a pH range of 2.5 to 11.5 under treatment conditions of 4°C and 20 hours;
(4) active temperature and optimum temperature: being active at least within a temperature range of 30 to 50°C, and being optimally active at about 45 to about 50°C;
(5) temperature stability: being stable at at least about 55°C under treatment conditions of pH 5 and 10 minutes;
(6) molecular weight: having an estimated molecular weight of about 27000 on SDS-PAGE;
(7) inhibitory properties: not being inhibited by 0.01 mg/ml SBTI but being inhibited by 1 mM PMSF and 0.1 mM DFP.

2. The protease according to claim 1, which is derived from a microorganism belonging to the genus *Fusarium.*

3. The following protein (a) or (b):
(a) a protein consisting of the amino acid sequence represented by SEQ ID NO: 1;
(b) a protein consisting of an amino acid sequence derived from the amino acid sequence represented by SEQ ID NO: 1 by deletion, substitution or addition of one or more amino acids, the protein being a protease having the properties (1) and (7) shown in claim 1.

4. A gene encoding the protein according to claim 3.

5. A gene consisting of the following DNA (i) or (ii):
(i) a DNA consisting of the nucleotide sequence represented by SEQ ID NO: 2;
(ii) a DNA that hybridizes, under stringent conditions, with a DNA consisting of a nucleotide sequence complementary to the DNA consisting of the nucleotide sequence represented by SEQ ID NO: 2, and that encodes a protein that is a protease having the properties (1) and (7) shown in claim 1.

6. A gene encoding any one of the following proteins (a), (b), and (c):
(a) a protein consisting of the amino acid sequence represented by SEQ ID NO: 1;
(b) a protein consisting of the amino acid sequence represented by SEQ ID NO: 1 wherein one or more amino acids have been deleted, substituted, or added, the protein being a protease having the properties (1) and (7) shown in claim 1;
(c) a protein having at least 80% homology with the amino acid sequence represented by SEQ ID NO: 1, the protein being a protease having the properties (1) and (7) shown in claim 1.

7. A recombinant vector containing a gene according to any one of claims 4 to 6.

8. A transformant containing the recombinant vector according to claim 7.

9. A process for producing a protease, the process comprising culturing the protease-producing microorganism according to claim 8 and recovering a protease from the culture medium.

10. A protease-producing microorganism that belongs to the genus *Fusarium* and that produces the protease according to claim 1.

11. The protease-producing microorganism according to claim 10, the microorganism being **characterized by**:
(iii) having an ITS-5.8S rDNA consisting of the nucleotide sequence represented by SEQ ID NO: 3 or a nucleotide sequence having at least 98% homology therewith; or
(iv) having a 28S rDNA consisting of the nucleotide sequence represented by SEQ ID NO: 4 or a nucleotide sequence having at least 98% homology therewith.

12. The protease-producing microorganism according to claim 10, which is *Fusarium* sp. strain BLB (FERM BP-10493).

13. A process for producing a protease, the process comprising culturing a protease-producing microorganism according to any one of claims 10 to 12 and recovering a protease from the culture medium.

14. A thrombolytic agent containing the protease according to claim 1 or the protein according to claim 3.

15. A method for treating or preventing thrombosis, comprising administering, to a thrombosis patient or a person who needs prophylactic treatment for thrombosis, the protease according to claim 1 or the protein according to claim 3, in an amount effective for treating or preventing thrombosis.

16. Use of the protease according to claim 1 or the protein according to claim 3 for the manufacture of a thrombolytic agent.

17. A food containing the protease according to claim 1 or the protein according to claim 3.

18. A fermented food obtained by inoculating a food material with a protease-producing microorganism according to any one of claims 10 to 12, and fermenting the food material.
